# EUROPEAN PATENT SPECIFICATION

(11) **EP 3 325 946 B1**
(45) Date of publication and mention of the grant of the patent: **09.12.2020**
(21) Application number: 16828691.2
(22) Date of filing: 25.07.2016
(51) Int. Cl.: G01N 21/47, G01N 21/17, G01N 21/41, G01N 21/55, G01N 33/48, G01N 33/53, G01N 21/45, G01N 33/543

(54) **COMPETITIVE SMALL MOLECULE DETECTION ASSAYS USING ARRAYED IMAGING REFLECTOMETRY**
KOMPETITIVE ASSAYS ZUR DETEKTION KLEINER MOLEKÜLE UNTER VERWENDUNG VON BILDGEBUNGSREFLEKTOMETRIE AN PROBENARRAYS
ESSAIS COMPÉTITIFS DE DÉTECTION DE PETITE MOLÉCULE PAR RÉFLECTOMÉTRIE D'IMAGERIE SUR DES TABLEAUX D'ÉCHANTILLONS

(30) Priority: 23.07.2015 US 201562196208 P
(43) Date of publication of application: 30.05.2018
(73) Proprietor: Adarza Biosystems, Inc., St. Peters, Missouri 63376 (US)
(72) Inventor: CARTER, Jared A., West Henrietta, NY 14586 (US); TRIPLETT, Emily J., West Henrietta, New York 14586 (US); STRIEMER, Christopher C., West Henrietta, New York 14586 (US); MILLER, Benjamin L., West Henrietta, New York 14586 (US)
(74) Representative: Greaves Brewster LLP
(86) International application number: PCT/US2016/043917
(87) International publication number: WO 2017/015669

(56) References cited:
- WO-A1-2014/012985
- US-A1- 2004 002 064
- US-A1- 2011 236 261
- US-A1- 2013 252 841
- US-A1- 2014 187 878
- JARED A CARTER ET AL: "Analysis of inflammatory biomarkers by Arrayed Imaging Reflectometry", BIOSENSORS AND BIOELECTRONICS, ELSEVIER SCIENCE LTD. UK, AMSTERDAM, NL, vol. 26, no. 9, 15 February 2011 (2011-02-15), pages 3944-3948, XP028200356, ISSN: 0956-5663, DOI: 10.1016/J.BIOS.2011.02.025 [retrieved on 2011-02-24]
- YING LI ET AL: "Recent developments and applications of surface plasmon resonance biosensors for the detection of mycotoxins in foodstuffs", FOOD CHEMISTRY, ELSEVIER LTD, NL, vol. 132, no. 3, 29 October 2011 (2011-10-29), pages 1549-1554, XP028450781, ISSN: 0308-8146, DOI: 10.1016/J.FOODCHEM.2011.10.109 [retrieved on 2011-11-20]
- LISA M. BONANNO ET AL: "Tunable Detection Sensitivity of Opiates in Urine via a Label-Free Porous Silicon Competitive Inhibition Immunosensor", ANALYTICAL CHEMISTRY, vol. 82, no. 2, 15 January 2010 (2010-01-15), pages 714-722, XP055526430, US ISSN: 0003-2700, DOI: 10.1021/ac902453h
- RASHMI SRIRAM ET AL: "Validation of Arrayed Imaging Reflectometry Biosensor Response for Protein-Antibody Interactions: Cross-Correlation of Theory, Experiment, and Complementary Techniques", ANALYTICAL CHEMISTRY, vol. 83, no. 10, 25 April 2011 (2011-04-25), pages 3750-3757, XP055526094, US ISSN: 0003-2700, DOI: 10.1021/ac2001302 & Rashmi Sriram ET AL: "Validation of arrayed imaging reflectometry biosensor response for protein-antibody interactions: cross-correlation of theory, experiment, and complementary techniques Supporting Information", Analytical chemistry, 15 May 2011 (2011-05-15), pages 3750-3757, XP055528710, United States DOI: 10.1021/ac2001302 Retrieved from the Internet: URL:https://pubs.acs.org/doi/suppl/10.1021 /ac2001302/suppl_file/ac2001302_si_001.pdf
- CHIU NAN-FU ET AL: "Evaluation of an affinity-amplified immunoassay of graphene oxide using surface plasmon resonance biosensors", VISUAL COMMUNICATIONS AND IMAGE PROCESSING; 20-1-2004 - 20-1-2004; SAN JOSE,, vol. 9506, 5 May 2015 (2015-05-05), pages 95061H-95061H, XP060053211, DOI: 10.1117/12.2182134 ISBN: 978-1-62841-730-2
- CARTER JARED A ET AL: "A label-free, multiplex competitive assay for small molecule pollutants", BIOSENSORS AND BIOELECTRONICS, ELSEVIER SCIENCE LTD. UK, AMSTERDAM, NL, vol. 77, 29 August 2015 (2015-08-29), pages 1-6, XP029311710, ISSN: 0956-5663, DOI: 10.1016/J.BIOS.2015.08.064
- Charles R Mace ET AL: "Theoretical and experimental analysis of arrayed imaging reflectometry as a sensitive proteomics technique", Analytical chemistry, 1 August 2006 (2006-08-01), pages 5578-5583, XP055357309, United States DOI: 10.1021/ac060473+ Retrieved from the Internet: URL:http://pubs.acs.org/doi/pdf/10.1021/ac 060473+

## Description

### FIELD OF THE INVENTION

The invention describes systems and methods of using arrayed imaging reflectometry for competitive small molecule detection assays.

### BACKGROUND

Human health concerns are driving an ever-increasing need for simple and sensitive methods for detecting a broad range of contaminants in the environment. Of particular interest are small molecules known or suspected to have deleterious health effects. While individual tests are available for some of these, there is no system available for detecting environmental pollutants in a label-free, multiplex fashion with high sensitivity and selectivity in human serum.

Jared A Carter et al: "Analysis of inflammatory biomarkers by Arrayed Imaging Reflectometry", Biosensors and Bioelectronics, Elsevier Ltd, vol. 26, no.9, 15 February 2011, pages 3944-3948 relates to the utility of Arrayed Imaging Reflectometry (AIR) microarrays for examining serum levels of inflammatory biomarkers.

Ying Li et al: "Recent developments and applications of surface plasmon resonance biosensors for the detection of mycotoxins in foodstuffs", Food Chemistry, Elsevier Ltd, vol. 132, no. 3, 29 October 2011, pages 1549-1554 relates to mycotoxin detection using SPR (Surface Plasmon Resonance) biosensor.

Lisa M. Bonanno et al: "Tunable detection sensitivity of opiates in urine via a label-free porous silicon competitive inhibition immunosensor", Analytical Chemistry, vol. 82, no.2, 15 January 2010, pages 714-722 discloses a chip-based label-free porous silicon (Psi) photonic sensor for detecting opiates in urine.

Rashmi Sriram et al: "Validation of arrayed imaging reflectometry biosensor response for protein-antibody interactions: cross correlation of theory, experiment, and complementary techniques", Analytical Chemistry, vol.83, no. 10, 25 April 2011, pages 3750-3757 relates to the comparison of a standard one-site Langmuir binding model describing probe-analyte interactions at the surface with the quantitative response of arrayed imaging reflectometry (AIR) to probe and analyte properties.

### SUMMARY

The present invention relates to arrays for small or large molecule detection and to corresponding methods as defined in the claims.

The present disclosure pertains to a novel biosensor format that enables rapid detection of multiple small molecule analytes using either competitive inhibition or competitive dissociation-style immunoassay format and a label-free optical detection modality for analyte quantification using the Arrayed Imaging Reflectometry platform (AIR). This methodology may be extended to a broad range of target molecules including drugs, drug metabolites, enzyme inhibitors, peptides and other molecules.

The present disclosure also pertains to a unified platform for simultaneous label-free multiplex assays for human proteins via direct immunoassay and small molecule pollutants via competitive immunoassay.

According to an example, a system for detecting small or large molecules using an arrayed imaging reflectometry (AIR) sensor chip is disclosed herein. The system includes an AIR sensor chip having a antireflective surface, an array including at least one probe solution deposited on the antireflective surface, and at least one blocking agent on the antireflective surface.

The present disclosure also relates to a method of preparing an arrayed imaging reflectometry (AIR) sensor chip for small or large molecule detection. The method includes printing an array of probes on the surface of the sensor chip, applying one or more blocking agents to the surface of the sensor chip, rinsing the sensor chip, and stabilizing the array on the sensor chip surface.

In yet another embodiment, an array for small or large molecule detection using an arrayed imaging reflectometry sensor chip, includes a probe printed on a surface of the sensor chip. The probe further includes a target molecule. When the sensor chip is contacted with a sample solution comprising an antibody and the target molecule, a portion of the antibody in the sample solution binds to the probe. An array signal measured using arrayed imaging reflectometry for the antibody engaged to the probe is compared to a standard response plot of a known series of target concentration AIR signals. When the array signal is fit to the plot of the known series of target concentration AIR signals, the amount of the target molecule in the sample solution is determined.

In one embodiment, an array for small or large molecule detection using an arrayed imaging reflectometry sensor chip includes a probe printed on the surface of the sensor chip. The probe includes a target molecule and an antibody engaged to the target molecule. When the sensor chip is contacted with a sample solution including a second target molecule, the antibody disassociates from the target molecule and binds to the second target molecule. An array signal is measured using arrayed imaging reflectometry after the disassociation and compared to a standard response plot of a known series of target concentration AIR signals to determine a level of disassociation for the antibody. When the array signal is fit to the plot of the known series of target concentration AIR signals, the amount of the target molecule in the sample solution is determined.

A method of detection using an arrayed imaging reflectometry (AIR) sensor chip includes providing an arrayed imaging reflectometry sensor chip and printing a of probe on a surface of the sensor chip. The probe includes at least one target molecule.

The method further includes contacting the sensor chip with a sample solution comprising an antibody and the target molecule so that a portion of the antibody in the sample solution binds to the probe. An array signal for the antibody engaged to the probe is measured using arrayed imaging reflectometry. The array signal for the antibody engaged to the probe is compared to a standard response plot of a known series of target concentration AIR signals. The amount of the target molecule in the sample solution is determined when the array signal is fit to the plot of the known series of target concentration AIR signals.

In yet another embodiment, a method of detecting small or large molecules using an arrayed imaging reflectometry (AIR) sensor chip includes providing an arrayed imaging reflectometry sensor chip and printing a probe on a surface of the sensor chip. The probe includes at least one target molecule and an antibody engaged to the target molecule. The method further includes contacting the sensor chip with a sample solution that includes a second target molecule; wherein the antibody disassociates from the target molecule and binds to the second target molecule. Next, the method includes measuring an array signal using arrayed imaging reflectometry after the disassociation and comparing the array signal to a standard response plot of a known series of target concentration AIR signals. Finally, a determination is made regarding the level of disassociation for the antibody and the amount of the target molecule in sample solution is determined when the array signal is fit to the plot of the known series of target concentration AIR signals.

### BRIEF DESCRIPTION OF DRAWINGS

FIG. 1 shows possible formats for competitive assays using the AIR platform.
FIG. 2 shows KLH conjugates of benzo[a]pyrene (1), bisphenol A (2), acrolein (3 and 4), and triclosan (5).
FIG. 3 illustrates verification of KLH-toxicant activity via standard ELISA.
FIG. 4 illustrates a competitive ELISA of anti bisphenol A.
FIG. 5 illustrates a representative KLH-Toxicant AIR array.
FIG. 6 illustrates a relative response for competitive assay formats.
FIG. 7 shows titration response profiles for three toxicants.
FIG. 8 shows the results of competitive assays.

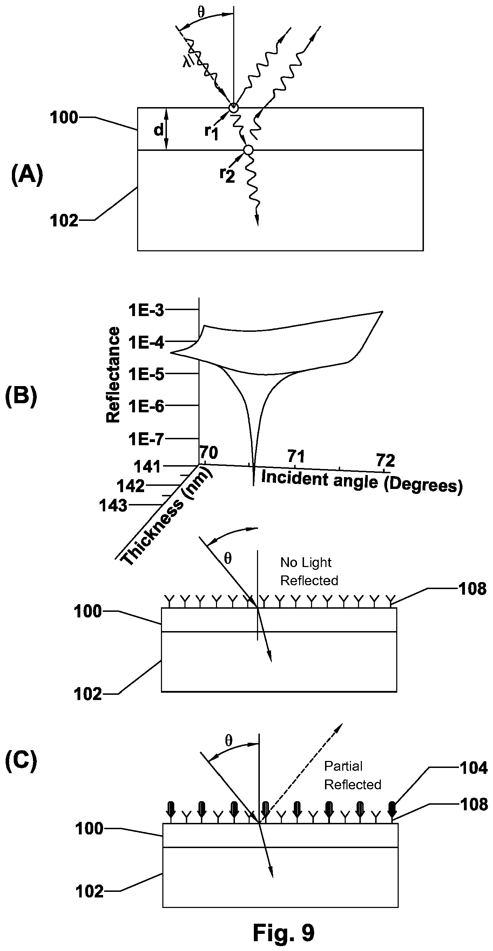

FIG. 9 shows a diagram of the implementation of AIR.
FIGS. 10A-E depict the results of using AIR for simultaneous detection of small molecules with competitive assays and protein markers with direct label-free detection.
FIGS. 11A-B show historical results of using AIR for direct label-free detection of protein markers.

### DETAILED DESCRIPTION

The present disclosure generally relates to a novel method for detecting small molecule analytes using the Arrayed Imaging Reflectometry (AIR) platform. These small molecule targets could include for example, but are not limited to, pollutants, drugs, drug metabolites, enzyme inhibitors, and peptides. Initially, small molecules are conjugated to a carrier protein because direct attachment of small molecules to a planar sensor surface may result in an inactive device due to the proximity to the surface acting as a steric barrier to antibody binding. As used herein, an antibody refers to a molecule that specifically binds a target molecule. Additionally, the term "antibody" includes any antibody including a monoclonal antibody. "Monoclonal antibody" refers to an antibody that is derived from a single copy or clone, including e.g., any eukaryotic, prokaryotic, or phage clone. "Monoclonal antibody" is not limited to antibodies produced through hybridoma technology. Monoclonal antibodies can be produced using e.g., hybridoma techniques well known in the art, as well as recombinant technologies, phage display technologies, synthetic technologies or combinations of such technologies and other technologies readily known in the art. Furthermore, the monoclonal antibody may be labeled with a detectable label, immobilized on a solid phase and/or conjugated with a heterologous compound (e.g., an enzyme or toxin) according to methods known in the art

The phrase "specifically binds" herein means antibodies bind to the analyte with an affinity constant or Affinity of interaction (KD) in the range of 0.1 pM to 1011M, with a preferred range being 0.1 pM to 1 nM. For purposes of this disclosure, keyhole limpet hemocyanin (KLH) was used as the carrier protein, however, the disclosed concept is not limited to use with KLH conjugates.

Details of the theoretical foundations and operation of AIR have been disclosed in related patents and applications. Additional features, techniques, and descriptions of the AIR technology are disclosed in U.S. Patent No. 7,292,349. In brief, the technique relies on the creation of a near-perfect antireflective condition on the surface of a silicon chip. When illuminated with S-polarized laser light at the HeNe wavelength and at an appropriate angle, an array of capture molecules spotted on the chip may be imaged with a CCD, showing minimal reflectivity in the absence of target. Binding of target analytes to the appropriate capture molecule spot causes a predictable, quantitative perturbation in the antireflective condition that may be measured as a change in reflected intensity. Thus far, it has been demonstrated that AIR is useful for detecting bacterial cell-surface proteins, human cytokines in serum, and a variety of immune system markers including antibodies to human papilloma virus and influenza. Quantitative analytical performance of AIR is well correlated with theory and reference techniques such as surface plasmon resonance (SPR) and spectroscopic ellipsometry.

Although AIR is capable of detecting small molecules directly, it is now being used to examine the performance benefits of a competitive assay format. This potentially allows for more sensitive detection of very small targets, effectively amplifying the amount of mass change observed in the sensor. Several examples of competitive assays in label-free sensor platforms have been reported. For example, publications have described a competitive format porous silicon sensor for urinary metabolites of morphine and related drugs of abuse. Two formats for such an assay are possible. In the competitive inhibition assay, a sensor surface is prepared with the target molecule covalently attached. Exposure of this sensor to a solution of the analyte of interest mixed with an appropriate antibody causes a loss of signal relative to that observed when the antibody alone is mixed with the sensor. Alternatively, in the competitive dissociation format, antibodies are pre-bound to the immobilized analytes on the sensor; the target analyte solution is then added. The competitive dissociation format has the advantage of providing a simpler work flow to the user; however, for this format to be successful, the binding affinities of surface-bound and solution-phase analytes must be comparable, and the surface-bound antigen-antibody complex must have a reasonable off-rate.

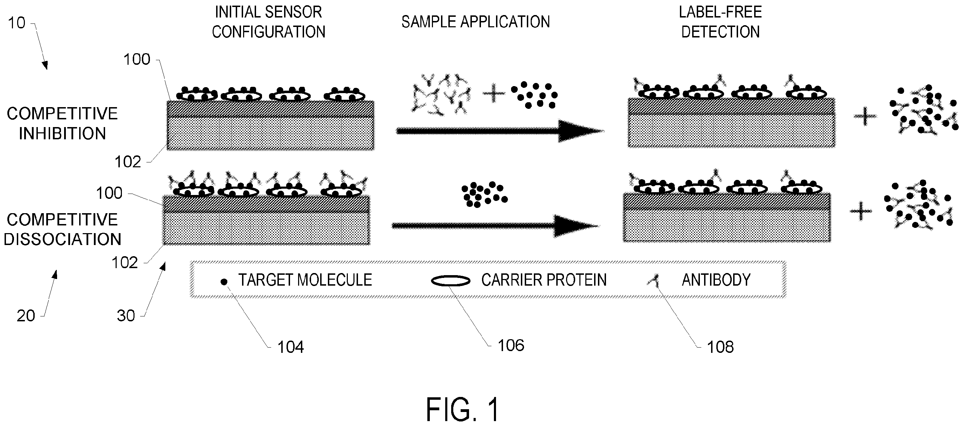

Referring now to Fig. 1, AIR assays are more sensitive if the starting conditions (control spots) are at or near the minimum reflectance condition. By way of example and not limitation, the fabrication of the assays begins by preparing chips 30, having a silicon dioxide coating 100 having a silicon substrate 102. The chips are cleaned and the thickness of the silicon dioxide coating 100 is made uniform across the chips. Alternatively, the silicon wafers may be manufactured having a uniform silicon dioxide coating prior to being cut into chips.

Next, an essential step in the fabrication of the toxicant array is to optimize printing conditions for conjugates that would yield uniform spots at the appropriate thickness. To prevent probe aggregation during spotting, a non-nucleophilic additive may be included. In some embodiments, passivation of remaining reactive groups on the surface of the chip was accomplished via immersion in a blocking solution. Chips to be used in assaying human serum samples may undergo a two-step blocking process to control array spot thickness.

These array chips can be used in either competitive inhibition assay, generally indicated as 10, or competitive dissociation assay, generally indicated as 20. These AIR toxicant arrays were able to selectively detect individual analytes doped in commercial pooled normal human serum (PNHS) in competitive inhibition experiments. Accordingly, this array is able to sensitively and specifically detect individual toxicants in relatively simple backgrounds and in human serum. AIR may be used for sensitive and specific detection of cytokines and other inflammatory biomarkers in serum. AIR chips may also be fabricated into a combined array able to simultaneously detect both a toxicant itself, and a cytokine-mediated inflammatory response.

In various embodiments, the AIR substrate may undergo a two-stop blocking process to control array spot thickness. The two step blocking process may be preceded by a spotting step, wherein the AIR substrate is spotted with one or more probe solutions.

In various embodiments the AIR substrates are spotted with probe solutions. Probe solution may be applied to the AIR substrates using s a variety of means. The probe solution may be manually applied. In other embodiments, probe solution is applied to the AIR substrate system using mechanical means. Mechanical means of applying probe solution may include, but not limited to the use of printers printing spots. In one preferred example, a Scienion SciFlex™Arrayer S3 piezoelectric printer equipped with a PDC50 capillary, or comparable device may be used to print spots without contacting the surface of substrate. In other embodiments, the probe solution may be applied using a Virtek ChipWriter™ Pro or comparable device using a wetted pin to contact the surface of substrate.

### Two-Step Blocking

In various embodiments, application of probe solution may be followed by two-step blocking. The two-stop blocking accomplishes several goals, one step provides a thickening layer on the surface of the AIR platform, and another blocking step creates an inert surface that is not prone to proteins or other molecules non-specifically binding to the AIR chip surface. These blocking steps mitigate the effects from interferences. The blocking may also make the chip surface resistant to fouling. In no particular order, the two step blocking may include exposing the chip to a first blocking solution and then exposing the chip to a second blocking solution. The first and second blocking solution may have different compositions. Alternatively, the first and second blocking solutions may have the same composition. Chip exposure to blocking solution may be accomplished by fully or partially immersing the chip into the solution. Alternatively, exposure may be accomplished by pouring solution onto the chip. In other embodiments, a chip may be exposed to blocking solution or buffer by spraying the buffer or solution on to the chip. The solution may be a fluid such as liquids, gas, plasmas, plastic solid suspension, or an emulsion that is fluid under ambient conditions. The solution also may have a solid form such as powder or other solid buffer solution form. One of skill in the art will appreciate that a chip may be exposed to solution by any means currently known in the art.

The chip may be exposed to the first or second solution for various amounts of time. In some embodiments the chip is exposed to the first and second blocking solutions for equal amounts of time. In other embodiments the chip is exposed to the first and second blocking solutions for different amounts of time. For a non-limiting example, the chip may be exposed to the first blocking solution for 20 minutes and the second blocking solution for 40 minutes. In other embodiments the chip may be exposed to the first or second blocking solution for a time between 1 second and 1 minute. In still other embodiments the chip may be exposed to a first or second blocking solution for a time between 1 minute and 20 minutes. In yet other embodiments, the chip may be exposed to the first or second solution for a time between 20 minutes and 1 hour. In still other embodiments, the chip may be exposed to the first or second solution for a time that is more than 1 hour.

In various embodiments, the AIR chip may be exposed to a blocking solution at a temperature of 4°C. In other embodiments the AIR chip may be exposed to a blocking solution at a temperature of more than 4°C. In other embodiments the AIR chip may be exposed to a blocking solution at a temperature of less than 4°C. One of skill in the art would appreciate that the AIR chip may be exposed to a blocking solution at any temperature known in the art for applying blocking agents.

The blocking solution may include any suitable blocking agent including but not limited to animal serum proteins, milk proteins, and fish serum proteins, non-animal serum proteins. Non-limiting examples of animal serum proteins include bovine serum albumin (BSA), newborn calf serum (NBCS), porcine serum, mouse, rat, fetal bovine serum, and goat serum. Casein is a non-limiting example of milk protein. Non-limiting examples of fish serum proteins include serum proteins derived from salmon or other fish species.

In various embodiments the first or second blocking solution may have various concentrations of blocking agents. In some embodiments, the blocking solution may include 0-10% blocking agent. In other embodiments, the blocking solution may include 10-20% blocking agent. In yet other embodiments, the blocking solution may include 20-50% blocking agent. In still other embodiments, the composition may include 50-100% blocking agent.

The first or second blocking solution may also include a buffer. In various embodiments the blocking solution may include any suitable buffer solution including but not limited to NaOAc solutions, PBS, PBS-ET, or other buffers currently known in the art.

In various embodiments, the blocking solution may have a pH off or about 5.0. In some embodiments, the blocking solution may have a pH off or about 7.4. In some embodiments, the blocking solution may have a pH between 5 and 6. In other embodiments, the blocking solution may have a pH from 6 to 7. In other embodiments, the blocking solution may have a pH from 7 to 8. In other embodiments, the blocking solution may have a pH from 4 to 5. In other embodiments, the blocking solution may have a pH from 8 to 10.

### Hapten conjugation

The AIR chip that has undergone the two step blocking process may be used for assays known in the art, including, but not limited to competitive inhibition assays and competitive dissociation assay. An advantage of the AIR chip is that it may be used as a label free detection platform operating as a direct assay.

In various embodiments the sensor surface of the AIR chip is prepared with a target molecule attached to the surface. The target molecule may be covalently attached to the surface. In some embodiments, the target molecule is immobilized on the sensor surface by cross-linkage, wherein the target molecules are bonded to one another forming a matrix on the chip surface. In other embodiment, target molecule may be immobilized on the chip surface. One of skill in the art will appreciate that more than one target molecule may be immobilized on the chip surface.

In various embodiments target molecules may be bound to a larger carrier. In one embodiment the target molecule is a hapten. Haptens may be conjugated by adding a linker to benzo (a)pyrene through Friedel-Crafts acylation. Hapten conjugation may further include equimolar quantities of benzo(a)pyrene and ethyl succinyl chloride being combined in the presence of two equivalents of AlC13 in dry dichloromethane under nitrogen. This reaction may be run under reflux. In various embodiments the reaction is monitored by thin layer chromatography. The product may then be quenched with ice and concentrated HCl. The product may then be washed with water. The product may then be dried over magnesium sulfate, concentrated with rotary evaporation, and stored at 4°C.

The benzo(a)pyrene-linker product and 4,4-bis(4-hydroxyphenyl) valeric acid (BHPVA) may be activated with 1.25 equivalents of 1-Ethyl-3-(3-dimethylaminopropyl)carbodiimide (EDC) and *N*-Hydroxysuccinimide (NHS) in Dimethylformamide (DMF) for 3 hours at room temperature at 400 rpm, before conjugation with keyhole limpet hemocyanin (KLH) at 2000-fold excess of small molecule to KLH in 100 mM sodium carbonate/bicarbonate buffer pH 10.0 for 20 hours at 4°C at 400 rpm, to form benzo(a)pyrene- and bisphenol A-KLH haptens. Acrolein 204 or 206 may be allowed to react with KLH under the same conditions to form the acrolein-KLH hapten. The reactions may then be quenched with 1% lysine and dialyzed against mPBS pH 6.0 with three buffer changes. One of skill in the art will appreciate that hapten conjugation may be accomplished by any means known in the art.

### Competitive inhibition

In embodiments where the AIR toxicant array may be used for competitive inhibition assay, a sensor surface is prepared with the target molecule attached. Exposure of the AIR chip to a solution of the analyte of interest mixed with an appropriate antibody causes a loss of signal relative to that observed when the antibody alone is mixed with the sensor. One of skill in the art will appreciate that the AIR array may be used for any competitive inhibition assay known in the art.

In various embodiments, dilutions of benzo[a]pyrene 200, bisphenol A, and acrolein 204 or 206 may be pre-incubated with the three respective antibodies. In one embodiment, the three antibodies are each presented at 1 µg/mL in 0.5% BSA in PBS-ET for one hour. In other embodiments, the concentration and time of incubation may be varied according to the desired assay performed.

Typically following hybridization, AIR substrates are then exposed to each solution for another hour. Following target exposure in each condition, the substrates are washed in PBS-ET and rinsed in purified water. The substrates are dried under a stream of nitrogen, and imaged. One of skill in the art will appreciate that the AIR array may be used for any competitive inhibition assay known in the art.

### Competitive Dissociation

In embodiments where the AIR toxicant array may be used for competitive dissociation format assays, antibodies are pre-bound to the immobilized analytes on the sensor; the target analyte solution is then added. The competitive dissociation format has the advantage of providing a simpler work flow to the user. In various embodiments the binding affinities of surface-bound and solution-phase analytes are comparable and the surface-bound antigen-antibody complex has a reasonable off-rate.

In various embodiments, substrate were exposed to a solution of the three antibodies (1 µg/mL each in PBS-ET plus 0.5% BSA) for one hour prior to exposure to a solution of 10µM benzo[a]pyrene 200, bisphenol A, and acrolein 204 or 206 in 0.5% BSA PBS-ET for another hour. Following target exposure in each condition, the substrates were washed in PBS-ET, rinsed in nanopure water or purified water, dried under a stream of nitrogen, and imaged. One of skill in the art will appreciate that the AIR array may be used for any competitive dissociation assay known in the art.

In various embodiments, the AIR platform may be used to detect a wide array of environmental toxicants. In other embodiments, the AIR platform may be used to detect toxicants in including environmental phenols, polycyclic aromatic hydrocarbons, and reactive aldehydes. The AIR platform may be fabricated so that a combined array is able to simultaneously detect both a toxicant itself, and a cytokine-mediated inflammatory response. In other embodiments, the AIR platform may also be configured to simultaneously detect proteins and small molecules in the same assay. One of skill in the art will understand that the AIR platform may be used to detect various analytes.

### Simultaneous Detection

In various embodiments a multiplex assay for common small molecule targets of toxicological studies is combined with a multiplex panel of human cytokines and inflammatory biomarkers to create a hybrid-small molecule and protein ("SMP") assay. In this respect, using the AIR chip, the multiplex assay is suitable for application in a wide range of application environments, producing robust data across analyte classes with simplified workflow and lower sample volume requirements. In some embodiments, obtaining all analyte concentrations requires <20 µL of serum.

In various embodiments, the sensors use 5 mm x 6 mm chips and only require sufficient sample to cover their surface. In prior work, <20 µL sample size has been used. In some embodiments, the sample may be blood. In some embodiments, the chip may measure chemicals or their metabolites at the level of ambient exposures as well as circulating proteins with appropriate limits of detection and specificity. One of skill in the art will appreciate that the sample may be urine, semen, cerebral spinal fluid, serum, or any other biological fluid. One of skill in the art will also appreciate that any sample may be used.

In various embodiments, environmental contaminant compounds and biological response indicators may be simultaneously profiled. In some embodiments, the hybrid-SW assay, using the AIR chip, accomplishes simultaneous profile on a single analytical platform.

### Example 1

*Sources of materials:* Irgasan (5-chloro-2(2,4-dichlorophenoxy)phenol), 4,4-bis(4-hydroxyphenyl) valeric acid (BHPVA), bisphenol A 202(BPA), benzo[a]pyrene 200, and N-hydroxysuccinimide (NHS) were obtained from Sigma-Aldrich (St. Louis, MO). Acrolein 204 or 206 was obtained from Ultra Scientific (N. Kingstown, RI), ethyl succinyl chloride and ethylenediaminetetraacetic acid from Acros Organics (Geel, Belgium), 6-chlorohexanoic acid from TCI Chemicals (Portland, OR), 1-ethyl-3-(3-dimethylaminopropyl)carbodiimide hydrochloride (EDC) from CreoSalus Life Sciences (Louisville, KY), Polysorbate 20 (Tween-20) from Avantor Performance Materials (Center Valley, PA), 3,3',5,5'-tetramethylbenzidine (TMB) from Alfa Aesar (Ward Hill, MA), bovine serum albumin (BSA) and peroxidase-conjugated protein A from Rockland Immunochemicals (Pottstown, PA), keyhole limpet hemocyanin (KLH) from EMD Millipore (Billerica, MA), porcine serum from Lampire Biologicals (Pipersville, PA), and human serum was obtained from Innovative Research (Novi, MI). Antibodies against benzo[a]pyrene 200 (GTX20768) and acrolein 204 or 206(GTX15138) were purchased from GeneTex (Irvine, CA). Anti-bisphenol A 202(AS132735) was obtained from Agrisera (Vannas, Sweden).

*Array fabrication:* Amine-reactive AIR substrates were spotted with probe solutions using a Scienion SciFlex™Arrayer S3 printer equipped with a PDC50 capillary. This provides non-contact, piezoelectric dispensing of 250 pL droplets, producing spots approximately 150 microns in diameter. All array spotting was conducted in a humidity-controlled chamber at 70% relative humidity. Following spotting, chips were immersed in a solution of 0.5% BSA in 50 mM NaOAc, pH 5.0 for 1 hour to block. Chips to be used in assaying human serum samples underwent a two-step blocking process, being first exposed to 0.5% BSA in NaOAc, pH 5.0 for 20 minutes, followed by exposure to a 1% porcine serum solution in PBS-ET, pH 7.4 for 40 minutes.

*Conjugation of haptens:* A linker was added to benzo(a)pyrene through a Friedel-Crafts acylation. Equimolar quantities of benzo(a)pyrene and ethyl succinyl chloride were combined in the presence of two equivalents of AlCl3 in dry dichloromethane under nitrogen. The reaction was run under reflux and monitored by thin layer chromatography. It was quenched with ice and concentrated HCl, and the product was washed with water, dried over magnesium sulfate, concentrated with rotary evaporation, and stored at 4°C.

The benzo[a]pyrene 200-linker product and 4,4-bis(4-hydroxyphenyl) valeric acid (BHPVA) were activated with 1.25 equivalents of EDC and NHS in DMF for (3 hours, room temperature, 400 rpm) before conjugation with keyhole limpet hemocyanin (KLH) at 2000-fold excess of small molecule to KLH in 100 mM sodium carbonate/bicarbonate buffer pH 10.0 (20 hours, 4°C, 400 rpm) to form benzo(a)pyrene- and bisphenol A-KLH haptens. Acrolein 204 or 206 was allowed to react with KLH under the same conditions to form the acrolein-KLH hapten. The reactions were quenched with 1% lysine and dialyzed against mPBS pH 6.0 with three buffer changes. The conjugations were confirmed through spectrophotometric analysis.

*Competitive binding experiments (AIR platform):* For the competitive inhibition experiments, dilutions of benzo(a)pyrene, bisphenol A, and acrolein 204 or 206 were pre-incubated with the three respective antibodies, each at 1µg/mL in 0.5% BSA in PBS-ET for one hour. Following hybridization, AIR substrates were exposed to each solution for another hour. For the competitive dissociation experiments substrate were exposed to a solution of the three antibodies (1µg/mL each in PBS-ET plus 0.5% BSA) for one hour prior to exposure to a solution of 10µM benzo[a]pyrene 200, bisphenol A, and acrolein 204 or 206 in 0.5% BSA PBS-ET for another hour. Following target exposure in each condition, the substrates were washed in PBS-ET, rinsed in nanopure water, dried under a stream of nitrogen, and imaged.

*Results:* Selected were four representative environmental toxicants of immediate interest to exposure biology in the US populace representing three classes of persistent organic pollutants, including environmental phenols (bisphenol A 202and triclosan 208), polycyclic aromatic hydrocarbons (benzo[a]pyrene 200)and reactive aldehydes (acrolein). These pollutants are currently subject to active monitoring by the CDC and were detected at biologically relevant concentrations in nearly all population substrata as described in the Fourth National Report on Human Exposure to Environmental Chemicals.

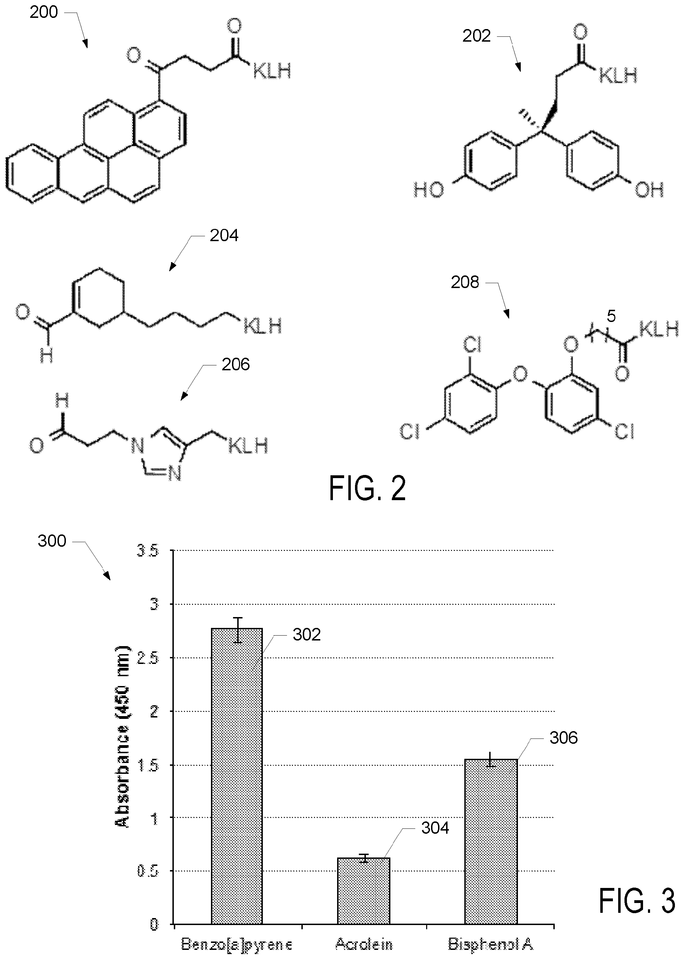

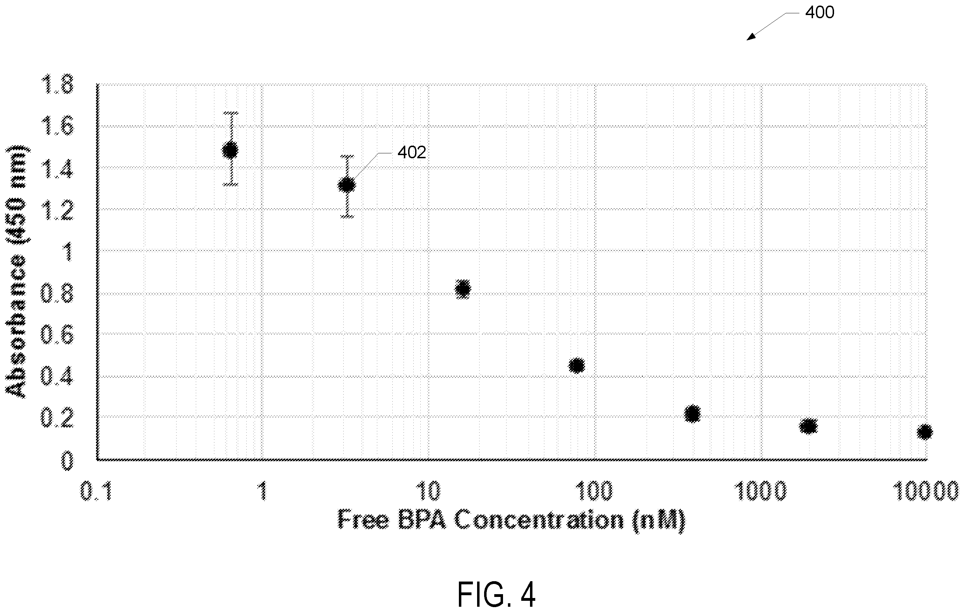

*Preparation of conjugates and confirmation of activity:* Direct attachment of small molecules to a planar sensor surface may result in an inactive device, since the proximity to the surface acts as a steric barrier to antibody binding. This can be addressed by first conjugating the small molecule 104 to a long-chain linker prior to immobilization; an alternative is to conjugate the small molecule 104 to a carrier protein 106, by analogy to standard methods used for raising antibodies 108 to small molecule targets. Since conjugate activity can vary depending on carrier protein 106, two possibilities were examined. Both bovine serum albumin (BSA) and keyhole limpet hemocyanin (KLH) are common carriers for hapten conjugation in antibody development, and were used here. Literature methods were employed for the preparation of protein conjugated analogs of bisphenol A, triclosan 208, acrolein, and benzo[a]pyrene 200. Initial experiments suggested that KLH conjugates had higher activity in our hands, and were used for all further experiments (Fig. 2). Characterization of KLH conjugates 302-306 to benzo[a]pyrene, acrolein, and bisphenol A are shown in the chart 300 in Fig. 3. The suitability of these constructs for incorporation into a competitive assay format was further tested via a competitive ELISA for bisphenol A 202 as a representative. Fig. 4 shows the competitive ELISA of anti bisphenol A, generally indicated as 400. In this assay, a styrene plate was physically adsorbed with KLH-bisphenol A, BSA-blocked, then exposed to anti-bisphenol A 202 either alone (positive control) or admixed with the various concentrations of free bisphenol A 202 as a competitor. Error bars indicate one sigma of the mean across triplicate spots on duplicate chips. The lower limit of detection for bisphenol A 202 in this format was 0.64 ng/mL (2.8 nM), indicated as 402 in Fig. 4. These data (not shown), indicated >95% specificity for each antibody to its antigen, yielding < 5% observable cross-reactivity in for all commercially sourced antibodies.

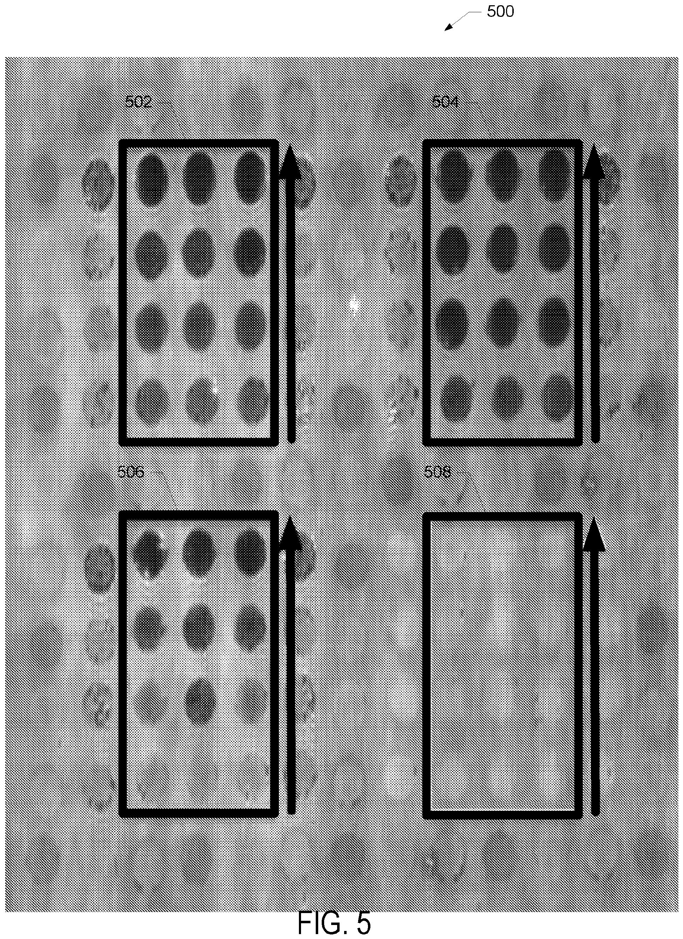

*Fabrication of the toxicant array:* AIR assays are most sensitive if the starting condition (e.g. control spots) are at or near the minimum reflectance condition. Therefore, an essential first step in the creation of the toxicant array was to determine printing conditions for conjugates that would yield uniform spots at the appropriate thickness. To that end, a concentration screening exercise was conducted to determine the optimal print concentration for each KLH-toxicant probe on the array. Additionally, as KLH is the carrier for all toxicants, dilutions of KLH were used, as well as other proteins to serve as nonreactive reference probes on the array to assist in normalization during downstream data analysis. A representative array 500 is shown in Fig. 5 (note that triclosan 208 conjugates were included in the experiment in anticipation of the availability of an effective triclosan 208 antibody). Conjugates of benzo[a]pyrene 502, acrolein 504, bisphenol A 506, and triclosan 508 are shown, where each row consists of three replicate spots. The concentration of the conjugate spotting solution in each row increases in the direction indicated by the arrow. For example, the concentrations for the spotting solution may be 0.5, 0.75, 1.0, and 1.5 mg/mL.

Aggregation in the control (KLH only) probes was observed, as evidenced by speckling in the spots. In one embodiment, a non-nucleophilic additive, such as but not limited to 5% DMSO was used to prevent aggregation during spotting. Passivation of the remaining reactive groups on the surface of the chip was accomplished via immersion in a blocking solution of 0.5% BSA in 50 mM NaOH (pH 5.0).

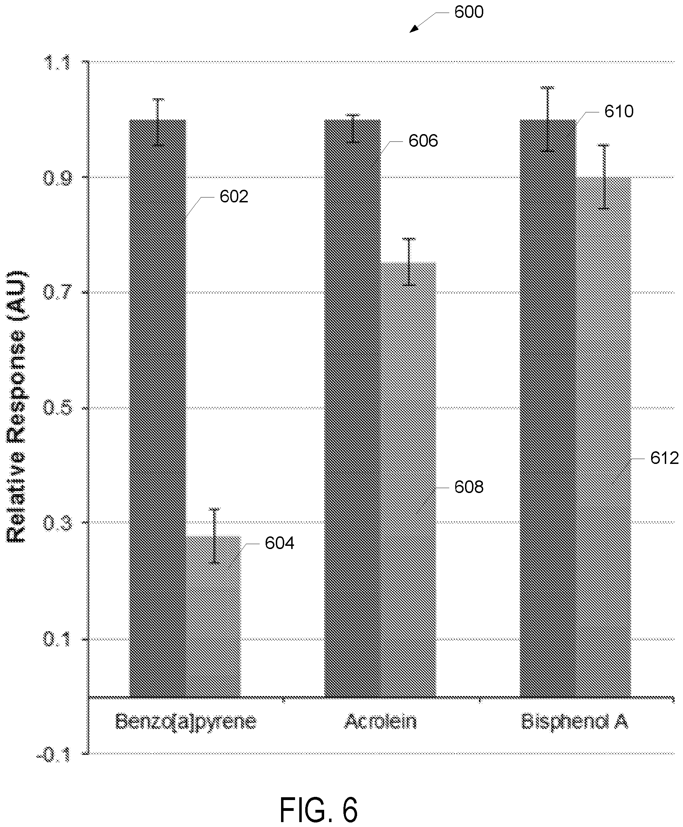

After determining the optimal parameters for the fabrication of the array, their use in the competitive inhibition assay 10 and competitive dissociation assay 20 formats was explored. It was observed that the competitive inhibition 10 format provided substantially greater response at equivalent concentrations for benzo[a]pyrene and acrolein, while activity in both formats was comparable for bisphenol A. Additional experiments were conducted in the competitive inhibition format. Fig. 6 shows a relative response 600 (where signal is scaled to greatest responder for each assay) for competitive inhibition assay formats, indicated as 602, 606, 610 and competitive dissociation assay formats, indicated as 604, 608, 612, implemented on the toxicant microarray. Error bars represent the standard deviation of mean probe response across three replicate sensor chips per condition.

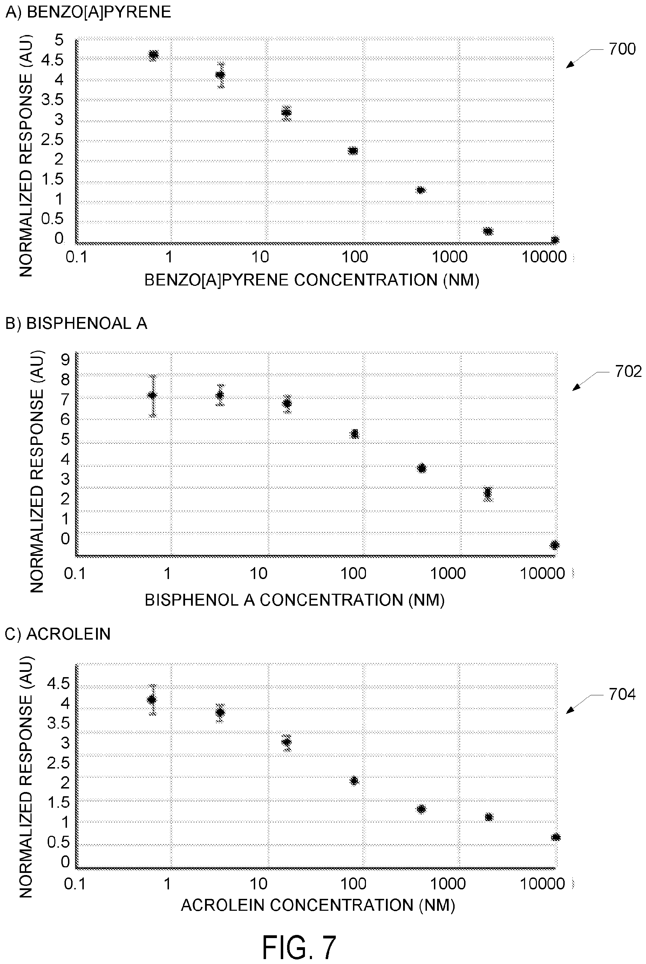

Response profiles for the three toxicants were next determined on the array, using a simple background matrix of 0.5% BSA in PBS-ET. In each case, the amount of the appropriate solution-phase antibody was kept constant at 6.7 nanomolar, while the concentration of analyte was varied from 10 micromolar to 640 pM in 1:4 serial dilutions. All three analytes produced well-behaved response curves. Fig. 7 shows titration response profiles 700-704 for benzo[a]pyrene (A), bisphenol A (B), and acrolein (C) in buffered 0.5% BSA, respectively. In each case, a constant amount of antibody was incubated with the array, while the concentration of the analyte was varied. Error bars represent the standard deviation of mean probe response across three replicate sensor chips per condition.

A lower limit of detection ("LLOD") for each assay was the lowest concentration at which a signal was observed that was greater than twice the signal error from the assay baseline. A lower limit of quantification ("LLOQ") for each assay was determined as the lowest concentration in the standard curve at which the coefficient of variation is less than 30% of the assay response. The coefficient of variation (CV) was determined by the relationship between the standard deviation of each dose dependent observation and its mean signal intensity, or more specifically CV = 100 ^{∗} (pt). the calculated LLOD for each analyte were consistent with concentrations observed in studies of human samples for all three analytes (benzo[a]pyrene, bisphenol A and acrolein).

**Table 1 provides the LLOD and LLOQ (nM) for each toxicant in the three-plex assay, according to one embodiment.**

| Analyte | LLOD | LLOQ |
|---|---|---|
| Benzo[a]pyrene | 16 | 16 |
| Acrolein | 16 | 16 |
| Bisphenol A | 80 | 80 |

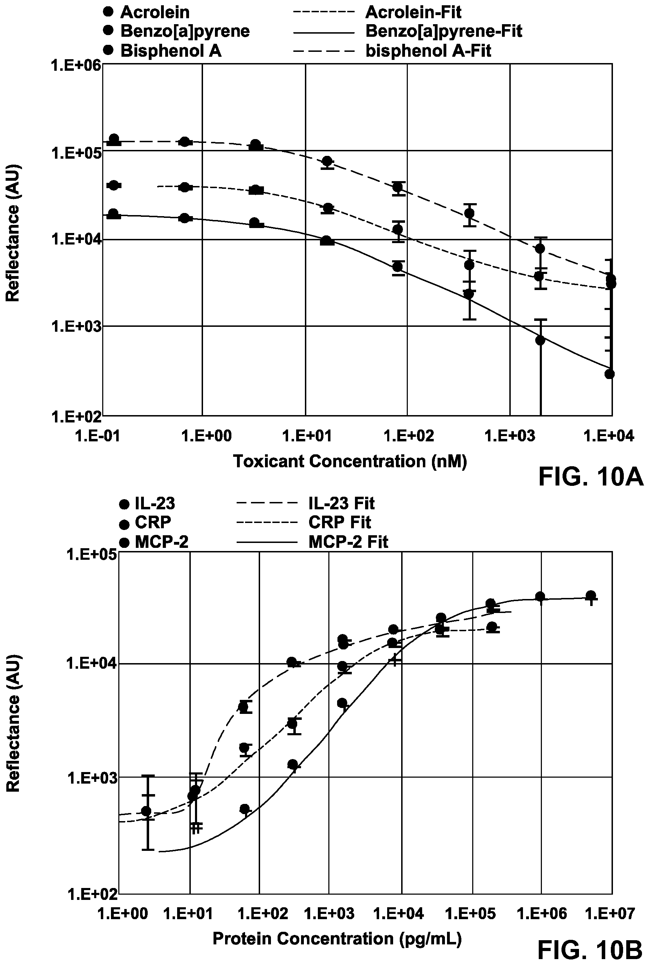

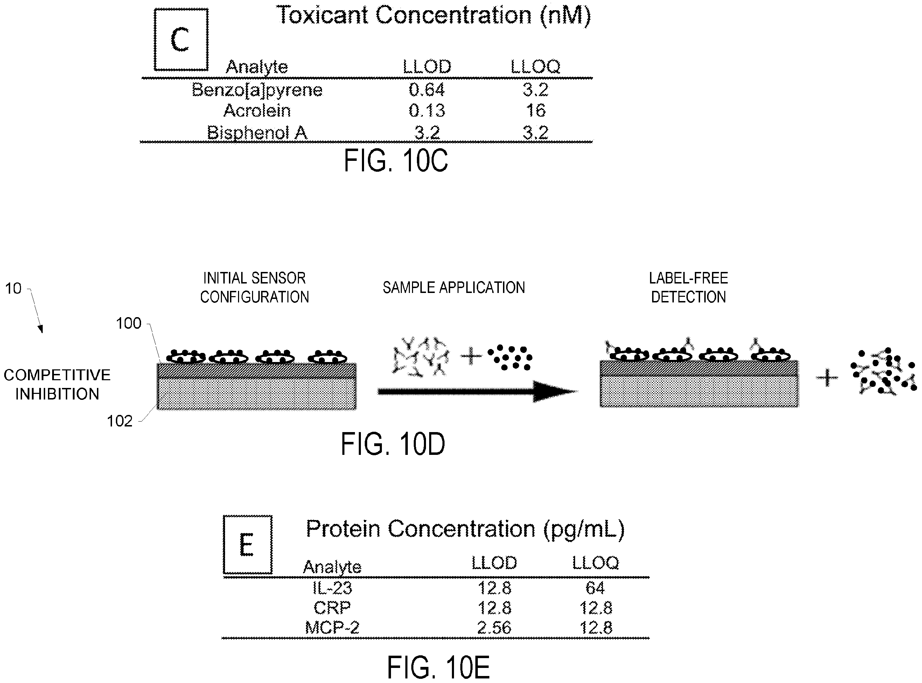

Improved results from subsequent studies using other embodiments were observed and are provided in Figs. 10A-C and E, explained more fully below.

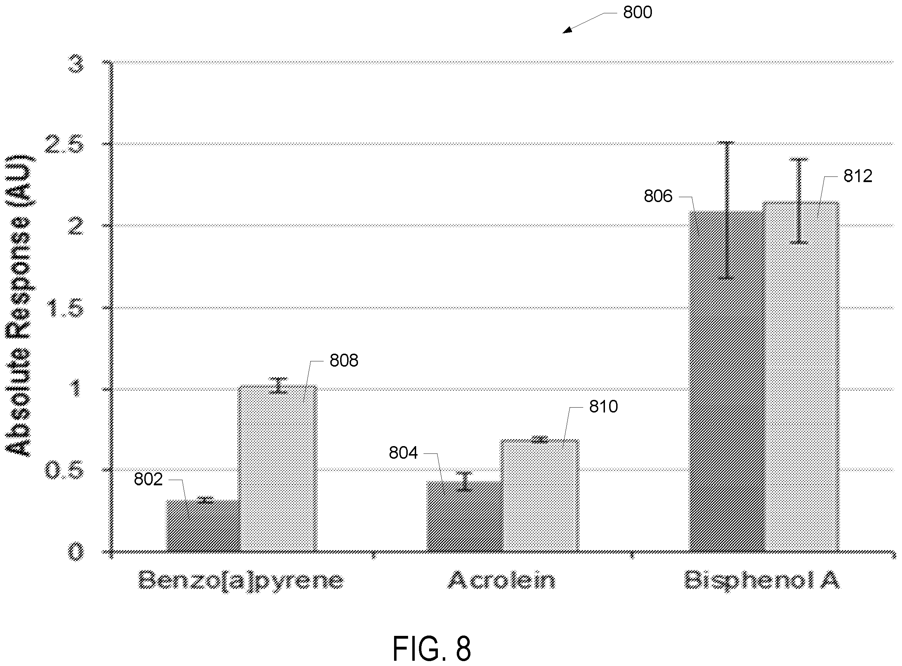

The array was also able to detect individual analytes doped in commercial pooled normal human serum (PNHS). Both benzo[a]pyrene 200 and acrolein 204 or 206 produced concentration-dependent responses at 10 and 50 micromolar; bisphenol A 202 produced a maximal response for both concentrations indicating some degree of nonspecific detection in the serum itself. Fig. 8 is an AIR toxicant array operating in competitive inhibition mode able to selectively detect three common toxicants in human serum Absolute responses 800 are shown for each analyte doped at 10 micromolar, indicated as 802, 804, and 806, and each analyte doped at 50 micromolar, indicated as 808, 810, 812. Error bars one sigma of the mean across triplicate spots on duplicate chips.

In another embodiment, a label-free, three-plex environmental toxicant array was used to sensitively and specifically detect benzo[a]pyrene 200, acrolein 204 or 206, and bisphenol A 202 in simple backgrounds and in human serum using a competitive immunoassay format. In various other aspects, a combined array able to simultaneously detect both the toxicant itself, and a cytokine-mediated inflammatory response may be used.

### Example 2

In this example protocols were developed to array conjugates on AIR substrates, and assays were tested individually in competitive inhibition and competitive dissociation formats. Competitive inhibition, shown in Fig. 10D was found to provide more robust detection of two targets, although both formats provided satisfactory data. The performance of the array with doped human serum samples was then tested. As shown in Fig. 10A, satisfactory dose-response curves were obtained for all three analytes printed on the array. Additionally, improved LLOD and LLOQ values were observed, as illustrated in Fig. 10C.

To further demonstrate the robustness of the AIR assay-format disclosed herein, antibodies specific to three serum inflammatory proteins were printed and assayed. Additionally, a cocktail consisting of six targets (three toxicants & three proteins) was also assayed in parallel. It is noted that the concentrations reported in Fig. 10A-E are the original concentrations in the serum samples.

According to various aspects and embodiments herein, the assays and associated AIR technology provide a novel multiplex label-free optical sensor able to directly measure common toxicants and serum inflammatory biomarkers in animal serum. By way of example and not limitation, the three toxicant assays demonstrate previously unknown limits of detection required to survey the reported plasma reference ranges for each toxicant. (e.g .Benzo[a]pyrene 200 at 0.95-5.2 nM, Acrolein 204 or 206 at 14,400-31,200 nM, Bisphenol A 202 at 2.8-12.7 nM).

Figs. 10A-B illustrate a simultaneous six-plex hybrid AIR toxicant array and inflammatory biomarker panel. The assay using the six-plex array generated normal, dose response profiles that are well-modeled by a standard 5-paramerter logistic function and yielded limits of detection and quantitation (in nM) for toxicants, shown in Fig. 10C and pg/mL for proteins of clinical relevance in Fig. 10E . The competitive assay mode used for the small molecules in this assay shown in Fig. 10D is similar to that shown in Fig. 1 for competitive inhibition. As such, this array provides two completely different assay formats (direct and competitive) being performed simultaneously on the same sensor substrate, and of simultaneous small molecule and cytokine detection.

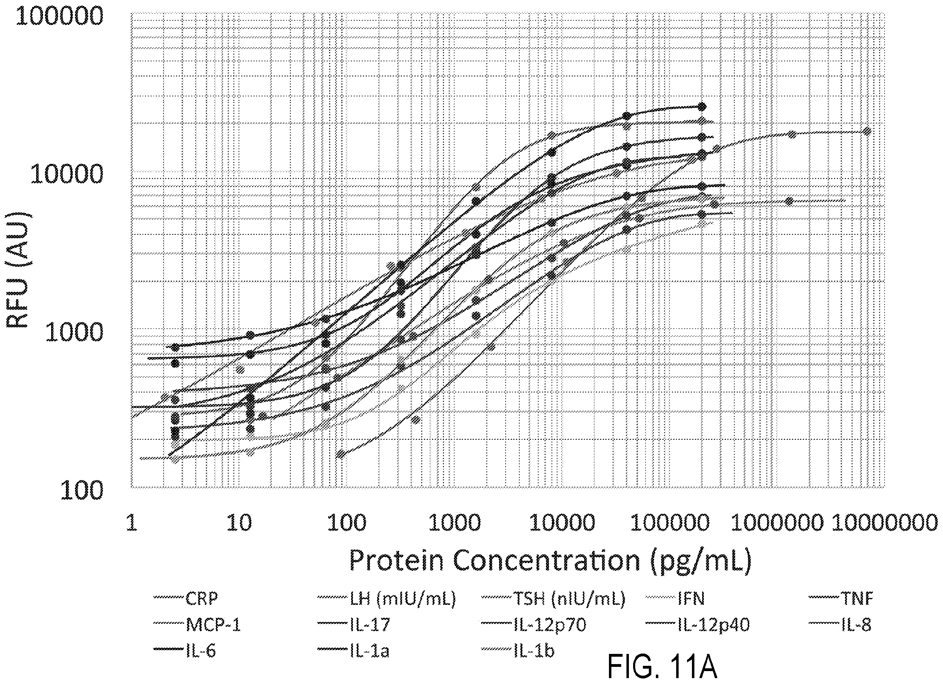

Figs. 11A-B illustrate the simultaneous detection of 13 serum biomarkers of general inflammation at physiologically relevant concentrations (LLOD as low as 1 pg/mL) using a single AIR biosensor fabricated with a highly sensitive array of antibody probes, according to embodiments disclosed herein. The standard curves, shown in Fig. 11A were generated using animal serum doped with example human proteins. The standard curves show correspond to measurements taken for the known presence of C-Reactive Protein ("CRP"), Thyroid Stimulating Hormone ("TSH"), Luteinizing Hormone ("LH"), Interleukin 1 alpha ("IL-1a"), Interleukin 1 beta ("IL-1b"), Interleukin 6 ("IL-6"), Interleukin 8 ("IL-8"), Interleukin 12 subunit p40 ("CR IL-12p40 P"), Interleukin 12 subunit p70 ("IL-12p70"), Interleukin 17 ("IL-17"), Interferon gamma ("IFN-g"), Monocyte Chemoattractant Protein-1("MCP-1"), Tumor Necrosis Factor alpha ("TNF-a"). Other large molecules, including but not limited to other protein biomarkers may also be identified by the assays and methods disclosed herein.

All serum samples were diluted 4:1 in a proprietary assay diluent containing proteins, surfactant, and blocking agents. The concentrations were normalized to reflect the original sample concentration, independent of dilution, for accurate benchmarking to standard assays. Additionally, this assay panel demonstrates the versatility of the AIR platform by enabling the detection of high concentration analytes (CRP), as well as low-abundance markers (cytokines) simultaneously on the same chip. The data further supports a key feature of the AIR technology and the competitive small molecule detection assays disclosed herein, which is allowing for the detection of serum protein markers from the low pg/mL to mid µg/mL range. This provides an effective dynamic range of approximately 7 logarithms.

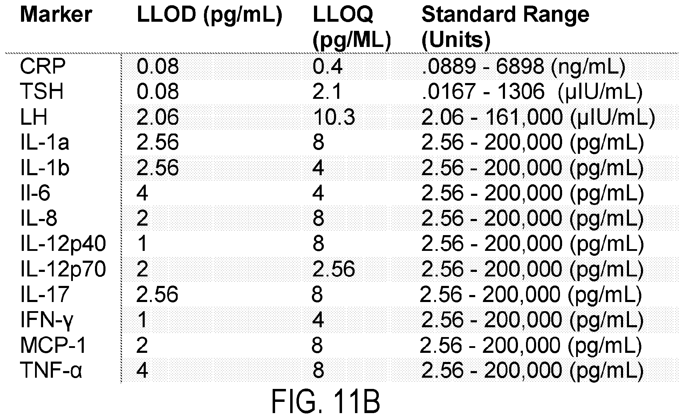

Figs. 11A-B also demonstrate that AIR technology, which can be used with various embodiments of the competitive assays 10 and 20, can capture and detect circulating protein biomarkers in human serum. As shown, the AIR antibody arrays show strong, titratable signals for their requisite antigen. The concentration shown in Fig. 11A for the CRP is ng/mL; µIU/mL for TSH and LH; and pg/mL for all others. These concentrations reflect the real concentration in the original samples. The chart shown in Fig. 11B provides data for the assay sensitivity (as LLOD) and detection performance (as LLOQ) is suitable for the surveillance of the baseline and elevated concentrations for all 13 assay constituents.

## Claims

1. An array for molecule detection using arrayed imaging reflectometry (AIR) on an arrayed imaging reflectometry (AIR) sensor chip (30), using a competitive inhibition style immunoassay, wherein the arrayed imaging reflectometry chip (30) has an antireflective surface, the array comprising:
a probe comprising a target molecule (104), printed on a surface of the sensor chip (30) wherein the target molecule is conjugated to a linker or bound to a carrier protein (106); and
a blocking agent, the blocking agent providing a thickening layer on the surface of the AIR sensor chip (30) and creating an inert surface that is not prone to proteins or other molecules non-specifically binding to the AIR sensor chip (30) surface; the AIR sensor chip (30) being configured such that when the AIR sensor chip (30) is contacted with a sample solution comprising the target molecule (104) and an antibody (108), that specifically binds to the target molecule (104), a portion of the antibody (108) binds to the probe.

2. An array for molecule detection using arrayed imaging reflectometry (AIR) on an arrayed imaging reflectometry (AIR) sensor chip (30), using a competitive dissociation style immunoassay, wherein the arrayed imaging reflectometry chip (30) has an antireflective surface, the array comprising:
a probe printed on the surface of the AIR sensor chip (30), the probe comprising a first target molecule (104) and a binding molecule (108) engaged to the first target molecule (104), wherein the first target molecule (104) is conjugated to a linker or bound to a carrier protein , (106) and the binding molecule (108) is an antibody; and
a blocking agent applied to the antireflective surface of the AIR sensor chip (30), the blocking agent providing a thickening layer on the surface of the AIR sensor chip (30) and creating an inert surface that is not prone to proteins or other molecules non-specifically binding to the AIR sensor chip (30) surface; the AIR chip (30) being configured such that when the AIR chip (30) is contacted with a sample solution comprising a second target molecule, the binding molecule (108) dissociates from the first target molecule (104) and binds to the second target molecule.

3. The array of claim 1, wherein the target molecule (104) of the probe is conjugated to a second target molecule

4. The array of any one of claims 1- 2, wherein the array comprises a plurality of probes.

5. The array of claim 2, wherein the array comprises a plurality of probes and wherein the plurality of probes comprise varied concentrations of the binding molecule (108).

6. A method of detection using an arrayed imaging reflectometry (AIR) sensor chip (30), the method comprising:
providing an arrayed imaging reflectometry sensor chip (30),
printing a probe on a surface of the AIR sensor chip (30), wherein the probe comprises at least one target molecule (104), wherein the target molecule (104) is conjugated to a linker or bound to a carrier protein (106);
contacting the AIR sensor chip (30) with a sample solution comprising the target molecule (104) and a binding molecule (108) that is an antibody that specifically binds to the target molecule (104) so that a portion of the binding molecule (108) in the sample solution binds to the probe;
measuring an array signal for the binding molecule (108) engaged to the probe using arrayed imaging reflectometry;
comparing the array signal for the binding molecule (108) engaged to the probe to a standard response plot of a known series of target concentration AIR signals; and determining the amount of the target molecule (104) in the sample solution when the array signal is fit to the plot of the known series of target concentration AIR signals.

7. The method of claim 6, wherein the target molecule (104) of the probe is conjugated to a second target molecule.

8. The method of claim 6 further comprising pre-incubating the binding molecule (108) and the target molecule (104) in the sample solution prior to contacting the AIR sensor chip (30).

9. The method of claim 6 wherein the probe is printed on an antireflective surface of the AIR sensor chip (30).

10. The method of claim 6, wherein the array signal measured using arrayed imaging reflectometry for the binding molecule (108) engaged to the probe is used to determine the concentration of the target molecule (104) in the sample solution.

11. A method of detecting small or large molecules using an arrayed imaging reflectometry (AIR) sensor chip (30), the method comprising:
providing an arrayed imaging reflectometry sensor chip (30),
printing a probe on a surface of the AIR sensor chip (30), wherein the probe comprises at least one first target molecule (104) and a binding molecule (108) engaged to the target molecule (104), wherein the target molecule (104) is conjugated to a linker or bound to a carrier protein (106) and wherein the binding molecule (108) is an antibody;
contacting the AIR sensor chip (30) with a sample solution comprising a second target molecule, wherein the binding molecule (108) disassociates from the first target molecule (104) and binds to the second target molecule;
measuring an array signal using arrayed imaging reflectometry after the disassociation;
comparing the array signal to a standard response plot of a known series of target concentration AIR signals;
determining a level of disassociation for the binding molecule (108);
determining the amount of the second target molecule in the sample solution when the array signal is fit to the plot of the known series of target concentration AIR signals.

## Patentansprüche

1. Array zur Molekül-Detektion unter Verwendung der Arrayed-Imaging-Reflektometrie (AIR) auf einem Arrayed-Imaging-Reflektometrie (AIR)-Sensorchip (30) unter Verwendung eines kompetitiven Immunoassays vom Inhibitionstyp, wobei der Arrayed-Imaging-Reflektometrie-Chip (30) eine Antireflexionsoberfläche aufweist, wobei das Array umfasst:
eine Sonde, die ein auf eine Oberfläche des Sensorchips (30) gedrucktes Zielmolekül (104) umfasst, wobei das Zielmolekül mit einem Linker konjugiert oder an ein Trägerprotein (106) gebunden ist, und
einen Blocker, wobei der Blocker eine Verdickungsschicht auf der Oberfläche des AIR-Sensorchips (30) bereitstellt und eine inerte Oberfläche bildet, die nicht anfällig ist für Proteine oder andere unspezifisch an die Oberfläche des AIR-Sensorchips (30) bindende Moleküle,
wobei der AIR-Sensorchip (30) so ausgebildet ist, dass, wenn der AIR-Sensorchip (30) mit einer das Zielmolekül (104) und einen Antikörper (108), der spezifisch an das Zielmolekül bindet, enthaltenden Probenlösung in Kontakt gebracht wird, ein Teil des Antikörpers (108) an die Sonde bindet.

2. Array zur Molekül-Detektion unter Verwendung der Arrayed-Imaging-Reflektometrie (AIR) auf einem Arrayed-Imaging-Reflektometrie (AIR)-Sensorchip (30) unter Verwendung eines kompetitiven Immunoassays vom Dissoziationstyp, wobei der Arrayed-Imaging-Reflektometrie-Chip (30) eine Antireflexionsoberfläche aufweist, wobei das Array umfasst:
eine auf die Oberfläche des AIR-Sensorchips (30) gedruckte Sonde, wobei die Sonde ein erstes Zielmolekül (104) und ein Bindungsmolekül (108) umfasst, das mit dem ersten Zielmolekül (104) verbunden ist, wobei das erste Zielmolekül (104) mit einem Linker konjugiert oder an ein Trägerprotein (106) gebunden ist und das Bindungsmolekül (108) ein Antikörper ist, und
einen Blocker, der auf die Antireflexionsoberfläche des AIR-Sensorchips (30) aufgetragen ist, wobei der Blocker eine Verdickungsschicht auf der Oberfläche des AIR-Sensorchips (30) bereitstellt und eine inerte Oberfläche bildet, die nicht anfällig ist für Proteine oder andere unspezifisch an die Oberfläche des AIR-Sensorchips (30) bindende Moleküle,
wobei der AIR-Sensorchip (30) so ausgebildet ist, dass, wenn der AIR-Sensorchip (30) mit einer ein zweites Zielmolekül enthaltenden Probenlösung in Kontakt gebracht wird, das Bindungsmolekül (108) von dem ersten Zielmolekül (104) dissoziiert und an das zweite Zielmolekül bindet.

3. Array nach Anspruch 1, wobei das Zielmolekül (104) der Sonde mit einem zweiten Zielmolekül konjugiert ist.

4. Array nach einem der Ansprüche 1 bis 2, wobei das Array eine Vielzahl von Sonden umfasst.

5. Array nach Anspruch 2, wobei das Array eine Vielzahl von Sonden umfasst und wobei die Vielzahl von Sonden veränderte Konzentrationen des Bindungsmoleküls (108) umfasst.

6. Detektionsverfahren unter Verwendung eines Arrayed-Imaging-Reflektometrie (AIR)-Sensorchips (30), wobei das Verfahren umfasst:
Bereitstellen eines Arrayed-Imaging-Reflektometrie (AIR)-Sensorchips (30),
Drucken einer Sonde auf eine Oberfläche des AIR-Sensorchips (30), wobei die Sonde wenigstens ein Zielmolekül (104) umfasst, wobei das Zielmolekül mit einem Linker konjugiert oder an ein Trägerprotein (106) gebunden ist,
Inkontaktbringen des AIR-Sensorchips (30) mit einer Probenlösung, die das Zielmolekül (104) und ein Bindungsmolekül (108) umfasst, das ein spezifisch an das Zielmolekül (104) bindender Antikörper ist, so dass ein Teil des Bindungsmoleküls (108) in der Probenlösung an die Sonde bindet,
Messen eines Array-Signals für das mit der Sonde verbundene Bindungsmolekül (108) unter Verwendung der Arrayed-Imaging-Reflektometrie,
Vergleichen des Array-Signals für das mit der Sonde verbundene Bindungsmolekül (108) mit einem Standard-Antwortdiagramm einer bekannten Reihe von Zielkonzentrations-AIR-Signalen und
Bestimmen der Menge des Zielmoleküls (104) in der Probenlösung, wenn das Array-Signal mit dem Diagramm der bekannten Reihe von Zielkonzentrations-AIR-Signalen abgeglichen wird.

7. Verfahren nach Anspruch 6, wobei das Zielmolekül (104) der Sonde mit einem zweiten Zielmolekül konjugiert ist.

8. Verfahren nach Anspruch 6, weiter umfassend das Vorinkubieren des Bindungsmoleküls (108) und des Zielmoleküls (104) in der Probenlösung vor dem Inkontaktbringen mit dem AIR-Sensorchip (30).

9. Verfahren nach Anspruch 6, wobei die Sonde auf eine Antireflexionsoberfläche des AIR-Sensorchips (30) gedruckt wird.

10. Verfahren nach Anspruch 6, wobei das Array-Signal, das für das mit der Sonde verbundene Bindungsmolekül (108) unter Verwendung der Arrayed-Imaging-Reflektometrie gemessen wird, verwendet wird, um die Konzentration des Zielmoleküls (104) in der Probenlösung zu bestimmen.

11. Verfahren zur Detektion kleiner oder großer Moleküle unter Verwendung eines Arrayed-Imaging-Reflektometrie (AIR)-Sensorchips (30), wobei das Verfahren umfasst:
Bereitstellen eines Arrayed-Imaging-Reflektometrie (AIR)-Sensorchips (30),
Drucken einer Sonde auf eine Oberfläche des AIR-Sensorchips (30), wobei die Sonde wenigstens ein erstes Zielmolekül (104) und ein Bindungsmolekül (108) umfasst, das mit dem Zielmolekül (104) verbunden ist, wobei das Zielmolekül (104) mit einem Linker konjugiert oder an ein Trägerprotein (106) gebunden ist und wobei das Bindungsmolekül (108) ein Antikörper ist,
Inkontaktbringen des AIR-Sensorchips (30) mit einer Probenlösung, die ein zweites Zielmolekül umfasst, wobei das Bindungsmolekül (108) von dem ersten Zielmolekül (104) dissoziiert und an das zweite Zielmolekül bindet,
Messen eines Array-Signals unter Verwendung der Arrayed-Imaging-Reflektometrie nach der Dissoziation,
Vergleichen des Array-Signals mit einem Standard-Antwortdiagramm einer bekannten Reihe von Zielkonzentrations-AIR-Signalen,
Bestimmen eines Dissoziationslevels für das Bindungsmolekül (108),
Bestimmen der Menge des zweiten Zielmoleküls in der Probenlösung, wenn das Array-Signal mit dem Diagramm der bekannten Reihe von Zielkonzentrations-AIR-Signalen abgeglichen wird.

## Revendications

1. Réseau destiné à la détection de molécules à l'aide d'une réflectométrie d'imagerie en réseau (AIR) sur une puce de capteur de réflectométrie d'imagerie en réseau (AIR) (30), à l'aide d'un essai immunologique de type inhibition compétitif, ladite puce de réflectométrie d'imagerie en réseau (30) possédant une surface antireflet, le réseau comprenant :
une sonde comprenant une molécule cible (104), imprimée sur une surface de la puce de capteur (30) ladite molécule cible étant conjuguée à un lieur ou liée à une protéine porteuse (106) ;
et un agent de blocage, l'agent de blocage fournissant une couche épaississante sur la surface de la puce de capteur d'AIR (30) et créant une surface inerte qui n'est pas sujette aux protéines ou autres molécules se liant de manière non spécifique à la surface de puce de capteur d'AIR (30) ; la puce de capteur d'AIR (30) étant conçu de sorte que lorsque la puce de capteur d'AIR (30) est mise en contact avec une solution échantillon comprenant la molécule cible (104) et un anticorps (108), qui se lie spécifiquement à la molécule cible (104), une partie de l'anticorps (108) se lie à la sonde.

2. Réseau destiné à la détection de molécules à l'aide d'une réflectométrie d'imagerie en réseau (AIR) sur une puce de capteur de réflectométrie d'imagerie en réseau (AIR) (30), à l'aide d'un essai immunologique de type dissociation compétitif, ladite puce de réflectométrie d'imagerie en réseau (30) possédant une surface antireflet, le réseau comprenant :
une sonde imprimée sur la surface de la puce de capteur d'AIR (30), la sonde comprenant une première molécule cible (104) et une molécule de liaison (108) en prise avec la première molécule cible (104), ladite première molécule cible (104) étant conjuguée à un lieur ou liée à une protéine porteuse (106) et ladite molécule de liaison (108) étant un anticorps ; et
un agent de blocage appliqué à la surface antireflet de la puce de capteur d'AIR (30), l'agent de blocage fournissant une couche épaississante sur la surface de la puce de capteur d'AIR (30) et créant une surface inerte qui n'est pas sujette aux protéines ou à d'autres molécules se liant de manière non spécifique à la surface de la puce de capteur d'AIR (30) ; la puce d'AIR (30) étant conçue de sorte que lorsque la puce d'AIR (30) est mise en contact avec une solution échantillon comprenant une seconde molécule cible, la molécule de liaison (108) se dissocie de la première molécule cible (104) et se lie à la seconde molécule cible.

3. Réseau selon la revendication 1, ladite molécule cible (104) de la sonde étant conjuguée à une seconde molécule cible.

4. Réseau selon l'une quelconque des revendications 1 à 2, ledit réseau comprenant une pluralité de sondes.

5. Réseau selon la revendication 2, ledit réseau comprenant une pluralité de sondes et ladite pluralité de sondes comprenant des concentrations variées de la molécule de liaison (108).

6. Procédé de détection à l'aide d'une puce de capteur à réflectométrie d'imagerie en réseau (AIR) (30), le procédé comprenant :
la fourniture d'une puce de capteur de réflectométrie d'imagerie en réseau (30), l'impression d'une sonde sur une surface de la puce de capteur d'AIR (30), ladite sonde comprenant au moins une molécule cible (104), ladite molécule cible (104) étant conjuguée à un lieur ou liée à une protéine porteuse (106) ;
la mise en contact de la puce de capteur d'AIR (30) avec une solution échantillon comprenant la molécule cible (104) et une molécule de liaison (108) qui est un anticorps qui se lie spécifiquement à la molécule cible (104) afin qu'une partie de la molécule de liaison (108) dans la solution échantillon se lie à la sonde ;
la mesure d'un signal de réseau pour la molécule de liaison (108) en prise avec la sonde à l'aide d'une réflectométrie d'imagerie en réseau ;
la comparaison du signal de réseau pour la molécule de liaison (108) en prise avec la sonde à un tracé de réponse standard d'une série connue de signaux d'AIR de concentration cible ; et la détermination de la quantité de la molécule cible (104) dans la solution échantillon lorsque le signal de réseau correspond au tracé de la série connue de signaux d'AIR de concentration cible.

7. Procédé selon la revendication 6, ladite molécule cible (104) de la sonde étant conjuguée à une seconde molécule cible.

8. Procédé selon la revendication 6, comprenant en outre la pré-incubation de la molécule de liaison (108) et de la molécule cible (104) dans la solution échantillon avant la mise en contact avec la puce de capteur d'AIR (30).

9. Procédé selon la revendication 6, ladite sonde étant imprimée sur une surface antireflet de la puce de capteur d'AIR (30).

10. Procédé selon la revendication 6, ledit signal de réseau mesuré à l'aide d'une réflectométrie d'imagerie en réseau pour la molécule de liaison (108) en prise avec la sonde étant utilisé pour déterminer la concentration de la molécule cible (104) dans la solution échantillon.

11. Procédé de détection de molécules petites ou grandes à l'aide d'une puce de capteur à réflectométrie d'imagerie en réseau (AIR) (30), le procédé comprenant :
la fourniture d'une puce de capteur de réflectométrie d'imagerie en réseau (30), l'impression d'une sonde sur une surface de la puce de capteur d'AIR (30), ladite sonde comprenant au moins une première molécule cible (104) et une molécule de liaison (108) en prise avec la molécule cible (104), ladite molécule cible (104) étant conjuguée à un lieur ou liée à une protéine porteuse (106) et ladite molécule de liaison (108) étant un anticorps ;
la mise en contact de la puce de capteur d'AIR (30) avec une solution échantillon comprenant une seconde molécule cible, ladite molécule de liaison (108) se dissociant de la première molécule cible (104) et se liant à la seconde molécule cible ;
la mesurer d'un signal de réseau à l'aide d'une réflectométrie d'imagerie en réseau après la dissociation ;
la comparaison du signal de réseau à un tracé de réponse standard d'une série connue de signaux d'AIR de concentration cible ;
la détermination d'un niveau de dissociation pour la molécule de liaison (108) ; la détermination de la quantité de la seconde molécule cible dans la solution échantillon lorsque le signal de réseau correspond au tracé de la série connue de signaux d'AIR de concentration cible.
